# EUROPEAN PATENT SPECIFICATION

(11) **EP 0 676 199 B1**
(45) Date of publication and mention of the grant of the patent: **02.09.1998**
(21) Application number: 95301959.3
(22) Date of filing: 23.03.1995
(51) Int. Cl.: A61K 31/435

(54) **Use of trovafloxacin or derivatives thereof for the manufacture of a medicament for the treatment of H. pylori infections**
Verwendung von Trovafloxacin oder dessen Derivaten zur Herstellung eines Arzneimittels zur Behandlung von H.Pylori Infektionen
Utilisation de trovafloxacine ou de ses dérivés pour la fabrication d'un médicament pour le traitement d'infections de H.pylori

(30) Priority: 07.04.1994 US 224392
(43) Date of publication of application: 11.10.1995
(73) Proprietor: PFIZER INC., New York, N.Y. 10017 (US)
(72) Inventor: Girard, Arthur E., Jewett City, Connecticut 06351 (US); Gootz, Thomas D., Deep River, Connecticut 06417 (US)
(74) Representative: Ruddock, Keith Stephen

(56) References cited:
- US-A- 5 164 402
- EUR.J.CLIN.MICROBIOL.INFECT.DIS., vol. 12, no. 2, 1993 pages 131-133, G.J.MALANOSKI ET AL. 'Effect of pH variation on the susceptibility of Helicobacter pylori to threee macrolide antimicrobial agents and temafloxacin'
- PATHOLOGIE BIOLOGIE, vol. 41, no. 4, 1993 pages 294-301, P. LE NOC ET AL. 'Activité antibactérienne comparée d'une nouvelle fluorquinolone, la sparfloxacine (AT 4140, RP 64206) et de 4 autres fluoroquinolones sur 332 souches de bactéries entéropathogènes'
- JOURNAL OF ANTIMICROBIAL CHEMOTHERAPY, vol. 22, no. 5, 1988 pages 631-636, DWIGHT J. HARDY ET AL. 'Susceptibility of Camphylobacter pylori to macrolides and fluoroquinolones'
- ANTIMICROBIAL AGENTS AND CHEMOTHERAPY, vol. 33, no. 1, 1989 pages 108-109, ANDREW E. SIMOR ET AL. 'Comparative in vitro activities of six new fluoroquinolones and other oral antimicrobial agents against Camphylobacter pylori'
- ZBL. BAKT., vol. 280, no. 1-2, 1993 pages 227-238, Y. GLUPCZYNSKI 'In vitro susceptibility testing of Helicobacter pylori to antimicrobial agents: Basis for treatment or microbiologists' obsession?'

## Description

This invention relates to a novel treatment of a Helicobacter infection with known naphthyridine carboxylic acid antibacterial compounds and a novel treatment of gastric and duodenal ulcers with such known compounds.

H.pylori, a pathogenic bacterium discovered in 1982, has been established as the cause of gastric and duodenal ulcers in humans. In clinical trials, eradication of the organism has been shown to result in healing of the ulcer and a low incidence of relapse. Although numerous individual antibacterial agents inhibit the growth of Helicobacter organisms in vitro, these agents usually fail in in vivo human and animal trials, when administered as single agents.

Infections in animals are caused by related organisms. For example, H. mustelae is pathogenic in ferrets, and H.felis in cats.

Eur.J.Microbiol.Infect.Dis., vol.12, no.2, 1993, pages 131-133, Pathol.Biol., vol.41, no.4, 1993, pages 294-301, J.Antimicrob. Chemother., vol.22, no.5, 1988, Antimicrob. Agents Chemother., vol.33, no.1, 1989 and Zbl. Bakt., vol. 280, no.1-2, 1993, describe the activity of certain fluoroquinolones against H. Pylori.

The compounds of use in this invention are disclosed in U.S.-A-5,164,402.

This invention relates to the use of a compound of the formula (I) (the active compound) or of a pharmaceutically acceptable acid addition salt or composition thereof for the manufacture of a medicament for the treatment of a Helicobacter pylori infection or gastric or duodenal ulcers:- 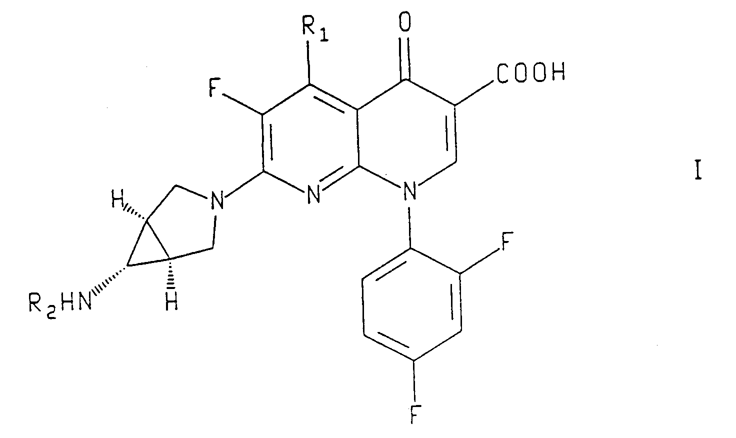 wherein R₁ is hydrogen or methyl and R₂ is hydrogen or L-alanine-L-alanyl.

In a specific embodiment of the invention, the active compound is 7-([1α, 5α, 6α]-6-amino-3-azabicyclo[3.1.0]hex-3-yl)-1-(2,4-difluorophenyl)-6-fluoro-1,4-dihydro-4-oxo-1,8-naphthyridine-3-carboxylic acid methanesulfonate.

The invention also relates to a method for the treatment of gastric and duodenal ulcers by administering to a subject in need of such treatment an effective amount of the active compound. In a specific embodiment, the compound used is 7-([1α, 5α, 6α]-6-amino-3-azabicyclo[3.1.0]hex-3-yl)-1-(2,4-difluorophenyl)-6-fluoro-1,4-dihydro-4-oxo-1,8-naphthyridine-3-carboxylic acid methanesulfonate.

The active compounds and their preparation are disclosed in U.S. Patent No. 5,164,402.

As disclosed in U.S. Patent No. 5,164,402, the compounds of formula I form pharmaceutically acceptable acid addition salts. All such salts are within the scope of this invention and can be prepared as described in U.S. Patent No. 5,164,402. Examples of suitable acids are acetic, lactic, succinic, maleic, tartaric, citric, gluconic, ascorbic, benzoic, methanesulfonic, p-toluenesulfonic, cinnamic, fumaric, phosphonic, hydrochloric, hydrobromic, hydroiodic, sulfamic, and sulfonic acid.

According to the invention, the active compound may be used in combination with a second antimicrobial agent, such as nitroimidazole antibiotics, e.g. tinidazole and metronidazole; tetracyclines, e.g. tetracycline, doxycycline and minocycline; penicillins, e.g. amoxicillin, ampicillin and mezlocillin; cephalosporins, e.g. cefaclor, cefadroxil, cephadrine, cefuroxime, cefuroxime axetil, cephalexin, cefpodoxime proxetil, ceftazidime and ceftriaxone; carbapenems, e.g. imipenem and meropenem; aminoglycosides, e.g. paromomycin; macrolide antibiotics, e.g. erythromycin, clarithromycin and azithromycin; lincosamide antibiotics, e.g. clindamycin; rifamycins, e.g. rifampicin; and nitrofurantoin. Also included within the invention are combinations of the active compound with a pharmaceutical compound used in the treatment of acid-related disorders such as acid pump inhibitors, e.g. omeprazole and lansoprazole, or H₂ antagonists, e.g. ranitidine, cimetidine, and famotidine.

A preferred combination according to the invention is 7-([1α,5α,6α]-6-amino-3-azabicyclo[3.1.0]hex-3-yl)-1-(2,4-difluorophenyl)-6-fluoro-1,4-dihydro-4-oxo-1,8-naphthyridine-3-carboxylic acid methanesulfonate and amoxicillin.

A second combination according to the invention is 7-([1α,5α,6α]-6-amino-3-azabicyclo[3.1.0]hex-3-yl)-1-(2,4-difluorophenyl)-6-fluoro-1,4-dihydro-4-oxo-1,8-naphthyridine-3-carboxylic acid methanesulfonate and tetracyclin.

A third preferred combination according to the invention is 7-[1α,5α,6α]-6-amino-3-azabicyclo[3.1.0]hex-3-yl)-1-(2,4-difluorophenyl)-6-fluoro-1,4-dihydro-4-oxo-1,8-naphthyridine-3-carboxylic acid methanesulfonate and omeprazole.

The active compounds are useful in the treatment of H. pylori in humans, and related Helicobacter infections in animals. They are also useful in the treatment of gastric and duodenal ulcers. As used herein, "treatment" is meant to include the eradication of the Helicobacter microorganism and the alleviation of the gastric and duodenal ulcers.

The active compounds of the invention may be administered alone, but will generally be administered in admixture with a pharmaceutical carrier selected with regard to the intended route of administration and standard pharmaceutical practice. For example, they can be administered orally or in the form of tablets containing such excipients as starch or lactose, or in capsules either alone or in admixture with excipients, or in the form of elixirs or suspensions containing flavoring or coloring agents. In the case of animals, they are advantageously contained in an animal feed or drinking water in a concentration of 5-5000 ppm, preferably 25-500 ppm.

The active compounds wherein R₂ is L-alanine-L-alanyl can be injected parenterally, for example, intramuscularly, intravenously or subcutaneously. For parenteral administration, they are best used in the form of a sterile aqueous solution which can contain other solutes, for example, enough salt or glucose to make the solution isotonic. In the case of animals, compounds can be administered intramuscularly or subcutaneously at dosage levels of about 0.1-50 mg/kg/day, advantageously 1-5 mg/kg/day given in a single daily dose or up to 3 divided doses.

The compounds of the invention can be administered to humans by either the oral or parenteral routes, and may be administered orally at dosage levels of about 0.1 to 50 mg/kg/day, advantageously 1-5 mg/kg/day given in a single dose or up to 3 divided doses. For intramuscular or intravenous administration, dosage levels are about 0.1-50 mg/kg/day, advantageously 1-5 mg/kg/day. While intramuscular administration may be a single dose or up to 3 divided doses, intravenous administration can include a continuous drip. Variations will necessarily occur depending on the weight and condition of the subject being treated and the particular routes of administration chosen as will be known to those skilled in the art.

The activity of the active compounds in vitro may be shown by the following procedure.

### Agar Dilution of Antimicrobial

6 mg. of the compound to be evaluated is solubilized in 0.6 ml 100% dimethylsulfoxide (DMSO) and then brought up to 6 ml with sterile brucella broth and the solubility is noted. The final concentration of DMSO is 10% of the total volume. Serial 2-fold dilutions (3 ml compound + 3 ml brucella broth) are then made in sterile brucella broth. A 2 ml aliquot of each broth dilution within the series is placed in separate sterile petri dishes, to which 18 ml of melted and cooled (approx. 50°C) brucella agar supplemented with 7% horse blood is added. This yields a final 1:10 dilution of compound in agar, and a final concentration of DMSO of 1%. For example, if the highest concentration (1st broth dilution) contains 1000 ug/ml, and is diluted 1:10 in agar, the final concentration of drug in agar is 100 ug/ml. Agar plates can be prepared one day prior to inoculating, and refrigerated overnight.

### Inocula Preparation

Helicobacter Pylori cultures are maintained on trypticase soy-5% sheep blood agar plates, and are transferred every 48 hours. Helicobacter mustelae cultures are maintained on the same agar, and are transferred every 48-60 hours, depending upon the extent of the growth of the previous transfer. Plates are incubated at 37°C in GasPak jars with water-activated (10 ml) CampyPak Plus (BBL Microbio. Systems) envelopes with palladium catalyst.

Helicobacter cultures can be grown in brucella broth supplemented with 10% fetal calf serum in 10 ml volumes in deep petri dishes. The plates are incubated for 18-20 hours at 37°C in GasPak jars with water-activated (10 ml) CampyPak Plus envelopes with palladium catalyst on a shaker at 50 rpm.

Overnight cultures (approx. 10⁸ CFU/ml) are diluted 10-fold in brucella broth (no FCS) in screw-capped tubes for use as the standard inoculum. The wells of a Steere replicator are filled with 0.8 ml of the diluted organism, and approximately 2 x 10⁴ cells in 0.002 ml are placed on the agar surface. Inoculated plates are placed in a GasPak jar to which water-activated (10 ml) Campy Pak Plus envelopes with palladium catalyst have been added, and incubated at 37°C for 48 hours.

### Interpretation of Results

Following incubation, all test plates are compared to a compound-free growth control plate. The MIC is the concentration which inhibits growth compared to the control plate. A thin film of growth might be visible at higher concentrations but this is discounted, and not considered the true MIC. Control organisms are also inoculated on each plate, and these are diluted 1000-fold for use as inocula. The control organisms include Campylobacter jejuni (#6686), and the screening cultures of E. coli (#51A266 and #51A470), Enterobacter aerogenes (#67A040), E. cloacae (#678009), Providencia stuartii (#77A013) and P. rettgeri (#77C025). Plates and/or inocula transfers should not be out of the microaerophilic environment longer than 2 hours. It is also recommended that all manipulations involving Helicobacter cultures be performed in a laminar flow hood to decrease the chance of contaminating the cultures with mold.

The mouse model of Lee et al., Gastroenterology, 99,1315-23(1990) is used to predict the in vivo activity of a compound against H. pylori in humans.

Helicobacter felis is grown in brucella broth with 10% fetal bovine serum. A frozen culture is quickly thawed; the culture is checked for motility and 0.5 cc. of the thawed frozen culture is inoculated into a deep tissue culture dish containing 9.5 cc. of the brucella/serum mix. The dishes are put into a Campy Pak jar [BBL] to insure a microaerophilic atmosphere. The jar is put on a rotary shaker at 60 RPM in a 37°C incubator. After 18 hours there should be visible turbidity. The culture is checked for purity and motility under a (phase) microscope and then pooled into a flask. Swiss-Webster female mice (18-20g) are fasted for 18 hours before infection. The mice are infected three times on alternate days during a single week. Dosing begins two weeks after the last dose of organism. Treatments are given once per day for fourteen consecutive days. Sacrifice is about three weeks after completion of therapy. For each mouse, the stomach is excised and opened along the greater curvature. A plug (a 3 mm. tissue section) is taken from the antrum region of the stomach. The plug surface is washed, minced, and dropped into a tube with 100 microliters of urease reagent. The urease reagent is the reagent of Hazell et. al., Am. J. Gastroenterology, 82, 292-296 (1982). The urease reagent (pH 6.3-6.5) contains urea and phenol red. If Helicobacter is present, urease will break down urea producing a change of pH. Purple (alkaline) is positive for Helicobacter; red/yellow (no change) is negative. Any color change is recorded after 18 hours. There are usually twenty mice per treatment group; the percent positive for each group is recorded.

There are several methods used clinically to determine whether Helicobacter pylori is present in a human subject. These are employed for initial diagnosis of infection prior to treatment, as well as for determining the success of treatment in eradicating the organism from the patient.

The urea breath test involves ingestion of radiolabelled urea. H. pylori produces a urease enzyme which degrades urea; mammalian gastric cells do not contain this enzyme. Exhalation of labeled carbon dioxide (analyzed by mass spectrometry or radioactivity, depending on the isotope employed) therefore indicates that H. pylori is present.

Serology can also be used to assess infection with H. pylori. Detection of serum antibodies to H. pylori, such as IgG and IgA, is carried out using enzyme-linked immunosorbent assay (ELISA). Numerous different H. pylori proteins can be employed as antigens.

Endoscopy of the patient provides samples of tissue which can be cultured in a microaerophilic environment to diagnose H. pylori infection. Alternatively, the sample can be examined histologically by employing one of a number of stains such as Giemsa or hematoxylin-eosin. A urea test, which again takes advantage of the production of urease by H. pylori, can also be applied. This test relies on the formation of ammonia from the urea hydrolysis, which results in an observable change in pH.

## Claims

1. The use of a compound of the formula 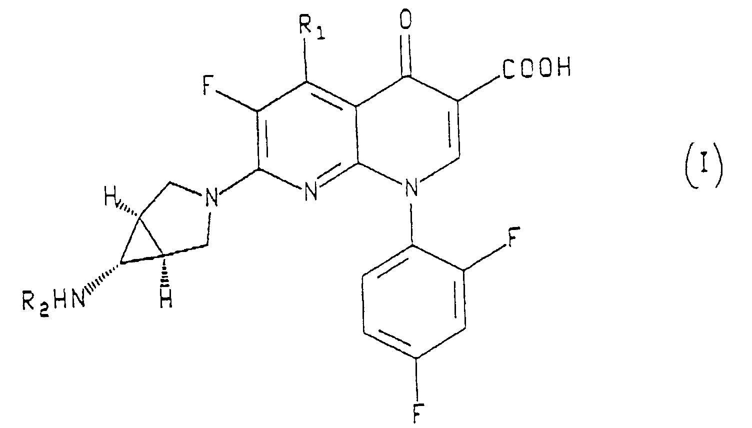 wherein R₁ is hydrogen or methyl and R₂ is hydrogen or L-alanine-L-alanyl, or a pharmaceutically acceptable acid addition salt or composition thereof, for the manufacture of a medicament for the treatment of (i) a Helicobacter pylori infection or (ii) gastric or duodenal ulcers.

2. Use according to claim 1 wherein said compound is 7-([1α, 5α, 6α]-6-amino-3-azabicyclo[3.1.0]hex-3-yl)-1-(2,4-difluorophenyl)-6-fluoro-1,4-dihydro-4-oxo-1,8-naphthyridine-3-carboxylic acid methanesulfonate.

3. Use according to claim 1 wherein R₂ is L-alanine-L-alanyl, and said compound, salt or composition is administered parenterally.

4. Use according to any one of claims 1 to 3 wherein said compound or salt is administered in combination with a second antimicrobial agent.

5. Use according to claim 4 wherein said second antimicrobial agent is amoxicillin.

6. Use according to claim 4 wherein said second antimicrobial agent is tetracycline.

7. Use according to claim 4 wherein the second antimicrobial agent is metronidazole.

8. Use according to any one of claims 1 to 3 wherein said compound or salt is administered in combination with a pharmaceutical compound for use in the treatment of acid-related disorders.

9. Use according to claim 8 wherein said compound is an acid pump inhibitor such as omeprazole.

## Patentansprüche

1. Verwendung einer Verbindung der Formel: 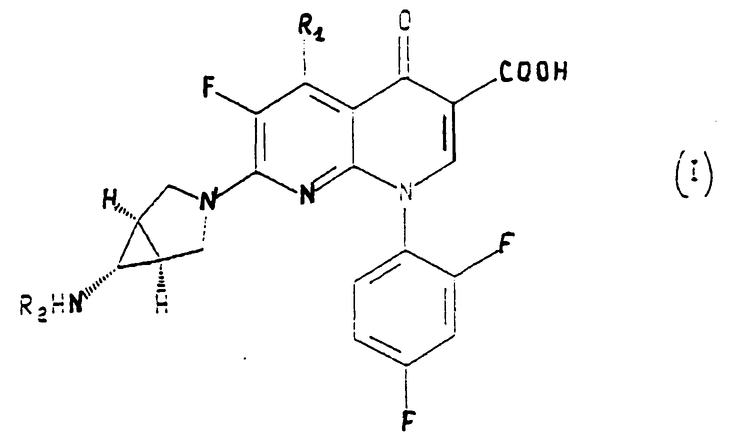 worin R₁ Wasserstoff oder ein Methylrest ist und R₂ Wasserstoff oder ein L-Alanin-L-alanylrest ist, oder eines pharmazeutisch annehmbaren Säureadditionssalzes oder einer Zusammensetzung davon zur Herstellung eines Arzneimittels zur Behandlung von (i) einer Helicobacter pylori-Infektion oder (ii) von Magen- oder Zwölffingerdarmgeschwüren.

2. Verwendung nach Anspruch 1, worin die Verbindung 7-([1α,5α,6α]-6-Amino-3-azabicyclo[3.1.0]hex-3-yl)-1-(2,4-difluorphenyl)-6-fluor-1,4-dihydro-4-oxo-1,8-naphthyridin-3-carbonsäuremethansulfonat ist.

3. Verwendung nach Anspruch 1, worin R₂ ein L-Alanin-L-alanylrest ist und die Verbindung, das Salz oder die Zusammensetzung parenteral verabreicht werden.

4. Verwendung nach einem der Ansprüche 1 bis 3, worin die Verbindung oder das Salz in Kombination mit einem zweiten antimikrobiellen Mittel verabreicht werden.

5. Verwendung nach Anspruch 4, worin das zweite antimikrobielle Mittel Amoxicillin ist.

6. Verwendung nach Anspruch 4, worin das zweite antimikrobielle Mittel Tetracyclin ist.

7. Verwendung nach Anspruch 4, worin das zweite antimikrobielle Mittel Metronidazol ist.

8. Verwendung nach einem der Ansprüch 1 bis 3, worin die Verbindung oder das Salz in Kombination mit einer pharmazeutischen Verbindung zur Verwendung zur Behandlung von mit Säure in Verbindung stehenden Störungen verabreicht wird.

9. Verwendung nach Anspruch 8, worin die Verbindung ein Inhibitor der Säurepumpe ist, wie Omeprazol.

## Revendications

1. Utilisation d'un composé de formule 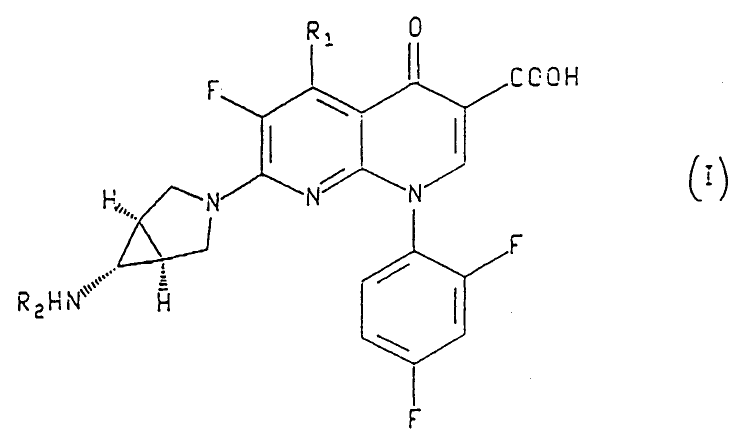 où R₁ est un hydrogène ou un méthyle et R₂ est un hydrogène ou un groupe L-alanine-L-alanyle, ou d'un de ses sels d'addition d'acide pharmaceutiquement acceptables, ou d'une composition en dérivant pour la fabrication d'un médicament destiné au traitement de (i) une infection par Helicobacter pylori ou (ii) des ulcères gastriques ou duodénaux.

2. Utilisation selon la revendication 1 dans laquelle ledit composé est le méthane sulfonate de l'acide 7-([1α, 5α, 6α]-6-amino-3-azabicyclo [3.1.0]hex-3-yl)-1-(2,4-difluorophényl)-6-fluoro-1,4-dihydro-4-oxo-1,8-naphtyridine-3-carboxylique.

3. Utilisation selon la revendication 1 dans laquelle R₂ est un L-alanine-L-alanyle, et ledit composé, sel ou composition est administré par voie parentérale.

4. Utilisation selon l'une quelconque des revendications 1 à 3, dans laquelle ledit composé ou sel est administré en combinaison avec un second agent antimicrobien.

5. Utilisation selon la revendication 4, dans laquelle ledit second agent antimicrobien est l'amoxicilline.

6. Utilisation selon la revendication 4, dans laquelle ledit second agent antimicrobien est la tétracycline.

7. Utilisation selon la revendication 4, dans laquelle ledit second agent antimicrobien est le métronidazole.

8. Utilisation selon l'une quelconque des revendications 1 à 3, dans laquelle ledit composé ou sel est administré en combinaison avec un composé pharmaceutique pour servir au traitement des désordres en relation avec l'acidité.

9. Utilisation selon la revendication 8, dans laquelle ledit composé est un inhibiteur de la pompe d'acide tel que l'oméprazole.
